# EUROPEAN PATENT SPECIFICATION

(11) **EP 2 888 036 B1**
(45) Date of publication and mention of the grant of the patent: **13.05.2020**
(21) Application number: 13830753.3
(22) Date of filing: 12.08.2013
(51) Int. Cl.: B01J 19/10, C07C 11/24, C07C 11/04, C07C 5/09, C07C 9/04, C01B 3/02

(54) **HIGH EFFICIENCY PROCESSES FOR OLEFINS, ALKYNES, AND HYDROGEN CO-PRODUCTION FROM LIGHT HYDROCARBONS SUCH AS METHANE**
HOCHEFFIZIENTE VERFAHREN ZUR GEMEINSAMEN HERSTELLUNG VON OLEFINEN, ALKYNEN UND WASSERSTOFF AUS LEICHTEN KOHLENWASSERSTOFFEN, Z. B. METHAN
PROCÉDÉS À HAUT RENDEMENT POUR LA COPRODUCTION D'OLÉFINES, D'ALCYNES ET D'HYDROGÈNE À PARTIR D'HYDROCARBURES LÉGERS TELS QUE LE MÉTHANE

(30) Priority: 21.08.2012 US 201261691369 P
(43) Date of publication of application: 01.07.2015
(73) Proprietor: UOP LLC, Des Plaines, Illinois 60017-5017 (US)
(72) Inventor: NEGIZ, Antoine, Des Plaines, Illinois 60017-5017 (US); JAMES, Robert B., Des Plaines, Illinois 60017-5017 (US); STEVENS, Carl J., Des Plaines, Illinois 60017-5017 (US); BARGER, Paul T., Des Plaines, Illinois 60017-5017 (US)
(74) Representative: Boult Wade Tennant LLP
(86) International application number: PCT/US2013/054469
(87) International publication number: WO 2014/031371

(56) References cited:
- WO-A1-2010/066281
- RU-C1- 2 145 952
- RU-C1- 2 158 747
- US-A- 2 958 716
- US-A- 4 724 272
- US-A1- 2009 042 998

## Description

### STATEMENT OF PRIORITY

This application claims priority to U.S. Application No. 61/691,369 which was filed on August 21, 2012.

### BACKGROUND OF THE INVENTION

The disclosure relates in general to producing alkenes, alkynes, and hydrogen using shockwave reactor technology. In certain embodiments, the disclosure relates to improving carbon efficiency using methanation techniques.

### DESCRIPTION OF THE RELATED ART

Converting light hydrocarbons such as methane to high value olefins such as ethylene is very economically attractive. However, in conventional pyrolysis processes, some of the feed methane is burned to achieve temperatures high enough to convert the methane, making the process require large amounts of light hydrocarbons, but yielding low carbon efficiency.

In conventional processes, methane can be converted to acetylene using either a one- or two-step process. An example of a one-step partial oxidation process developed by BASF is described in U.S. Patent Nos. 5,824,834 and 5,789,644. The general reactor configuration and design is described in U.S. Patent No. 5,789,644. Acetylene can also be produced using two-stage high temperature pyrolysis and an example two stage reactor developed by HOECHST is described in Great Britain Patent Application Publication Nos. GB 921,305 and GB 958,046.

In conventional processes, an air separation unit can be used to separate oxygen from nitrogen. The oxygen or an oxygen containing stream, along with natural gas (composed primarily of methane) are preheated and enter a partial oxidation reactor. In the BASF one stage reactor the hydrocarbon feed and oxygen rich gas are mixed and passed through a burner block which is used to stabilize the flame that results in partial oxidation of the mixture. Secondary oxygen can be injected at the burner block to create pilot flames. The burning converts one-third of the methane to acetylene, while most of the remainder is used to produce heat and lower valued products such as CO and CO₂. The residence time required for the reaction process is less than 100 milliseconds. In the two stage reactor, natural gas or other fuel are mixed with an oxygen rich stream and burned in a combustion zone. The combustion products are then mixed with feedstock consisting of natural gas or other hydrocarbons which react to form acetylene. Again, a reaction time of less than 100 milliseconds is used. After the desired residence time, the reacting gas is quenched with water. The cooled gas contains large amounts of carbon monoxide and hydrogen as well as some carbon soot, carbon dioxide, acetylene, methane, and other gases.

Next, the gas passes through a water scrubber to remove the carbon soot. The gas then passes through a second scrubber in which the gas is sprayed with a solvent, such as N-methylpyrrolidinone, which absorbs the acetylene.

The solvent is then pumped into a separation tower and the acetylene is boiled out of the solvent and removed at the top of the tower as a gas, while the solvent is drawn out of the bottom.

The acetylene can be used to make a variety of useful products. One such product is ethylene, which can be produced by catalytically hydrogenating acetylene. A process for hydrogenating acetylene to ethylene in the presence of a Pd/A12O3 catalyst is described in U.S. Patent No. 5,847,250. A process for hydrogenating acetylene over a palladium-based catalyst using a liquid solvent, such as N-methylpyrrolidinone, is described in U.S. Patent Application Publication Nos. 2005/0048658 and 2005/0049445.

Other known processes for converting methane to ethylene can be found in U.S. Patent No. 7,208,647 to Synfuels International.

Burning methane to generate heat for the pyrolysis reaction consumes carbon, which limits the amount of methane that can be converted to acetylene. As such, technology to improve carbon efficiency is desired.

US 4,724,272 describes a method of controlling temperature during a pyrolysis reaction wherein the predominant pyrolysis reactions are endothermic.

### SUMMARY OF THE INVENTION

The invention consists in a method of making alkenes and alkynes, as defined in claim 1, and further detailed by the depending claims.

In one aspect, the invention provides a method of making alkenes and alkynes. The method includes the steps of: combusting a fuel and an oxidizer in a combustion zone of a pyrolytic reactor to create a combustion gas stream; transitioning a velocity of the combustion gas stream from subsonic to supersonic in an expansion zone of the pyrolytic reactor; injecting a light hydrocarbon into the supersonic combustion gas stream to create a mixed stream including the light hydrocarbon; transitioning the velocity of the mixed stream from supersonic to subsonic in a reaction zone of the pyrolytic reactor to produce an alkyne; and catalytically hydrogenating the alkyne in a hydrogenation zone to produce an alkene. In one embodiment, the fuel is hydrogen, the oxidizer is oxygen, the light hydrocarbon is methane, the alkyne is acetylene, and the alkene is ethylene.

In another aspect, the invention provides a method of making alkenes and alkynes. The method includes the steps of: performing pyrolysis of a light hydrocarbon in the presence of oxygen in a reaction zone at a temperature and pressure suitable to produce an alkyne and carbon monoxide; catalytically hydrogenating the alkyne in a hydrogenation zone to produce an alkene; directing the carbon monoxide to a CO shift device; converting at least a portion of the carbon monoxide to hydrogen in the CO shift device to produce a stream including the hydrogen; and directing the stream including the hydrogen into the reaction zone.

In yet another aspect, the invention provides a method of making alkenes and alkynes. The method includes the steps of: performing pyrolysis of a light hydrocarbon in the presence of oxygen in a reaction zone at a temperature and pressure suitable to produce an alkyne and carbon dioxide; catalytically hydrogenating the alkyne in a hydrogenation zone to produce an alkene; converting at least a portion of the carbon dioxide to methane in a carbon dioxide conversion and methanation zone; and directing a stream including the methane from the carbon dioxide conversion and methanation zone into the reaction zone.

It is therefore an advantage of the invention to provide a process for converting light hydrocarbons such as methane to high value olefins such as ethylene that is more carbon efficient and environmentally friendly.

It is another advantage of the invention to provide a shock wave reactor that can operate at very high temperature and millisecond range very small residence times, which increases the overall C₂ selectivity with respect to the methane converted.

It is yet another advantage of the invention to provide various process configurations for producing ethylene and on-demand hydrogen with very high carbon efficiencies and very low CO₂ emissions.

It is still another advantage of the invention to provide various process configurations for producing ethylene from methane wherein the burning of methane is minimized.

It is yet another advantage of the invention to provide a process for converting light hydrocarbons such as methane to high value olefins such as ethylene wherein the process has better carbon utilization efficiencies and product selectivities to ethylene (hence acetylene) with respect to the methane feedstock.

It is still another advantage of the invention to provide various process configurations for producing ethylene from methane wherein less ethane is produced.

These and other features, aspects, and advantages of the present invention will become better understood upon consideration of the following detailed description, drawings and appended claims.

### BRIEF DESCRIPTION OF THE DRAWINGS

Figure 1 is a longitudinal cross section of an example pyrolytic reactor that can be used in processes according to the invention.
Figure 2 is a schematic process flow diagram of one process according to the invention for converting methane to ethylene.
Figure 3 is a schematic process flow diagram of another process according to the invention for converting methane to ethylene.
Figure 4 is a schematic process flow diagram of yet another process according to the invention for converting methane to ethylene.
Figure 5 is a schematic process flow diagram of still another process according to the invention for converting methane to ethylene.

Like reference numerals will be used to refer to like parts from Figure to Figure in the following description of the drawings.

### DETAILED DESCRIPTION OF THE INVENTION

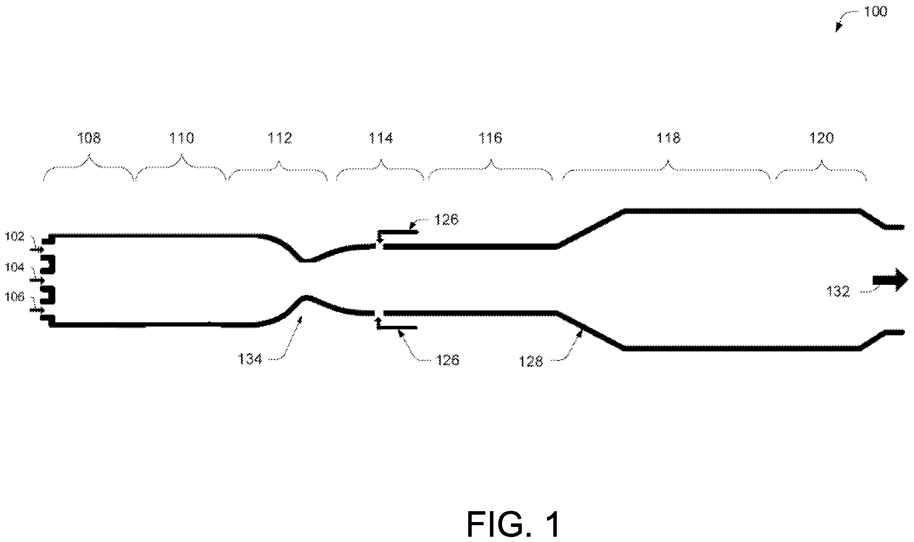

Turning to Figure 1, the conversion of methane to acetylene can be accomplished by thermal processing using a pyrolytic reactor 100. The methane feedstock is heated to a temperature at which the formation of acetylene is thermodynamically favored over that of methane. Additional energy must be provided to the reaction mixture to satisfy the endothermic reaction for the formation of acetylene. After a residence time sufficient to result in the desired acetylene formation, the reaction mixture is quickly quenched to freeze the reaction in order to prevent the acetylene from cracking into hydrogen and carbon and reforming as methane. A fuel and oxidizer are combusted to create a high temperature (e.g., >1500 K) and high speed (e.g., > Mach 1) combustion gas, in order to favor acetylene formation. Next, a sufficient amount of reaction enthalpy is provided to satisfy the 377 kJ/mol required for the formation of acetylene. If additional energy is not provided, the endothermic nature of the acetylene formation may drive the temperature below 1500 K. Finally, the reaction mixture is quickly cooled at a rate faster than the rate at which the acetylene can decompose into hydrogen and carbon and subsequently reform as methane. This quick cooling process is sometimes referred to as "freezing" the reaction when the amount of acetylene is high. It is desirable to initiate the freezing step at the stage of maximum acetylene formation (i.e., the point of thermodynamic equilibrium) and to complete the freezing step as quickly as possible to prevent the decomposition of any acetylene.

Still referring to Figure 1, a longitudinal cross section of an exemplary pyrolytic reactor 100 is depicted. In one embodiment, the reactor 100 is tubular (i.e., the transverse cross section is circular). The high temperatures necessary for the formation of acetylene as well as controlled residence time and rapid quenching can be achieved in the pyrolytic reactor 100. Fuel 102 and an oxidizer 106 are injected in the fuel injection zone 108 at the proximal end of reactor 100. In one embodiment the fuel and oxygen are heated to a temperature of 400° to 800°C, or to a temperature of 200° to 1000°C in another embodiment. In one example embodiment, the fuel is hydrogen, the oxidizer is oxygen, and the ratio of hydrogen to oxygen is a 3/1 molar ratio.

In some embodiments, the fuel 102 and oxidizer 106 are mixed prior to injection into the fuel injection zone 108. In some embodiments, the fuel 102 and oxidizer 106 are injected into the fuel injection zone 108 and mixed by the turbulent conditions within the fuel injection zone 108. In some embodiments, steam or other diluents 104 is also injected into the fuel injection zone 108.

The fuel and oxidizer are combusted in the combustion zone 110. The resulting combustion gas stream is heated to a high temperature by the combustion reaction. In some embodiments, the temperature of the combustion gas stream is 2500 K to 3500 K in the combustion zone 110. In other embodiments, the temperature of the combustion gas stream reaches is 2000 K to 4000 K in the combustion zone 110.

The combustion zone is operated at a pressure of 2 to 10 bar in one embodiment. In other embodiments the combustion zone 110 is operated at a pressure of 1.2 bar to 20 bar. The pressure within the combustion zone 110 propels the combustion gas stream toward the distal end of the reactor 100 at high velocity. In some embodiments, the velocity of the combustion gas stream at the distal end of the combustion zone 110 is below supersonic speed (i.e., less than Mach 1).

The subsonic combustion gas stream enters the expansion zone 112 and flows through a convergent-divergent nozzle 134. The convergent-divergent nozzle 134 transforms a portion of the thermal energy in the combustion gas stream into kinetic energy, resulting in a sharp increase in velocity of the combustion gas stream. The velocity of the combustion gas stream transitions from subsonic (i.e., less than Mach 1) to supersonic (i.e., greater than Mach 1) within the expansion zone 112. In one embodiment, at the distal end of the expansion zone 112, the temperature of the combustion gas stream is 2000 K to 3000 K. In one embodiment, at the distal end of the expansion zone 112, the average velocity of the combustion gas stream (across a transverse cross section) is greater than Mach 1. In one embodiment, the average velocity of the combustion gas stream is Mach 2 or above.

Feedstock is injected into the supersonic combustion gas stream in the feedstock injection zone 114. In one embodiment, the feedstock is injected at a temperature of 700 K to 1200 K. In one embodiment feedstock is injected at a temperature of 300 K to 2000 K. In one embodiment, feed lines 126 supply the feedstock. In one embodiment designed to remove impurities such as sulfur and chloride species, natural gas is mixed with a hydrogen containing stream to produce a stream with 0 to 5 mol% hydrogen (or more) and heated to 370°C and fed to a set of swing reactors that contains a hydrodesulfurization catalyst (e.g. CoMo on Alumina) and an H₂S adsorbent (e.g. ZnO) downstream of the hydrogenation catalyst either in the same vessel or in a different vessel. The H₂S resulting from hydrodesulfurization will react with the adsorbent. The same system will remove organic chlorides present in the natural gas feed. The reactor that is offline can be regenerated by methods known in the art for example by using air or steam. If the natural gas contains high levels of H₂S (for example higher than 20 ppm) another embodiment would be to treat the natural gas with known gas sweetening processes such as membrane processes, solvent absorption with chemical or physical solvents in order to lower the H₂S content of the natural gas to levels that are economical for the hydrosulfurization/adsorbent system.

The combined stream composed of the combustion gas stream and the feedstock stream enters mixing zone 116 where the combined stream is mixed as a result of the turbulent flow in the stream. In one embodiment oblique or normal shockwaves can be used to assist the mixing.

In one embodiment the transverse cross section of the reactor 100 increases in the reactor zone 118 due to an angled wall 128. As the mixed stream enters the reactor zone 118 and expands into the larger area, this results in a decrease in velocity of the mixed stream.

In some embodiments, the velocity of the mixed stream remains at supersonic velocities within the reaction zone 118. The reduction in velocity of the combined stream converts a portion of the kinetic energy of the combined stream into thermal energy. The combined stream is then reduced to subsonic flow and quenched in quenching zone 120.

In some embodiments, the velocity of the mixed stream transitions from supersonic to subsonic within the reaction zone 118. At this transition point, a shockwave is formed, which results in a nearly instantaneous increase in the pressure and temperature of the mixed stream. In various embodiments, the temperature of the mixed stream immediately upstream of the shock wave is 1500 K to 2300 K, as compared to 1600 K to 2800 K immediately downstream of the shockwave. The conditions in the mixed stream downstream of the shockwave are favorable to the formation of acetylene. Thus, the pyrolytic reactor 100 can be called a shock wave reactor (SWR).

In some embodiments, a shock train is formed at the point where the stream transitions from supersonic to subsonic flow. A shock train is a series of weak shock waves that propagate downstream from the supersonic to subsonic transition point. Whereas a single shockwave will heat the mixture nearly instantaneously (at the location of the shockwave), a shock train will heat the mixture more gradually. Each shock wave in the shock train will increase the temperature of the stream.

The mixed stream is increased to a temperature sufficient to favor the formation of acetylene and to provide enough energy to satisfy the endothermic reaction.

In one embodiment, the product stream exits the reaction zone 118 and enters the quenching zone 120 to rapidly cool the product stream. In one embodiment, the quenching zone 118 comprises at least one injection nozzle to spray the product stream with water. The product stream is removed at location 132.

In order to maintain steady state operation of the reactor 100 over a long period of time, the combustion zone 110 can be cooled. For example, a cooling jacket can be disposed over the reactor wall near the combustion zone 110, thereby forming a coolant channel. A coolant, such as water, can be introduced into the coolant channel. In one embodiment, the coolant flows in a direction opposite to that of the combustion gas stream in the reactor. The coolant effluent flows out of the coolant channel at an outlet.

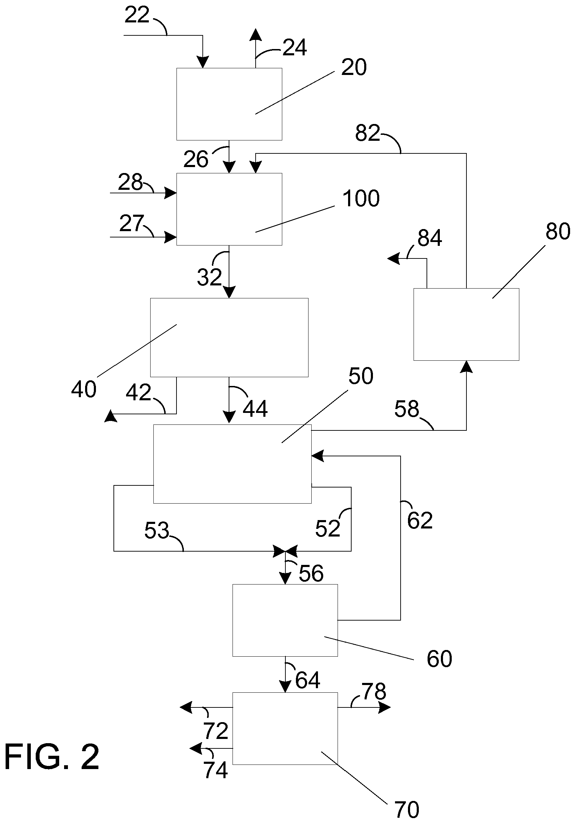

Turning now to Figure 2, there is shown an example process according to the invention for converting a light hydrocarbon (e.g., methane) to an alkyne (e.g., acetylene) and then converting the alkyne (e.g., acetylene) to an olefin (e.g., ethylene). First, the air separation unit 20 extracts oxygen from the air. The air separation unit 20 receives air via the air line 22, and generates the nitrogen rich stream 24 in which the oxygen content is less than that of air. The nitrogen rich stream 24 can be vented or reused. The air separation unit 20 also generates the oxygen rich stream 26 in which the oxygen content is greater than that of air. The air separation unit 20 can use processes known in the art such as cryogenic separation, membranes, or a pressure swing adsorption (PSA) process. In other embodiments an oxygen containing stream 26 can be obtained from pipeline or other sources.

In the example process of Figure 2, a hydrocarbon feedstock is converted into acetylene in the pyrolytic reactor 100 (SWR) of Figure 1. In one non-limiting example the hydrocarbon feedstock is methane. The pyrolytic reactor 100 receives methane (CH₄) via feed lines 126 (see Fig. 1) that receive methane from methane line 28. The pyrolytic reactor 100 receives the oxidizer (oxygen) via oxygen rich stream 26. The pyrolytic reactor 100 receives the fuel (hydrogen) via hydrogen stream 27. A pyrolytic reactor outlet stream 32 produced by the pyrolytic reactor 100 may include acetylene, ethylene, hydrogen, methane, carbon monoxide, carbon dioxide, and carbon particulates.

The pyrolytic reactor outlet stream 32 is fed into the quench unit 40 to rapidly cool the reactive mixture in the pyrolytic reactor outlet stream 32. The quench unit 40 may be a separate unit, or it may be incorporated into the quenching zone 120 (see Fig. 1) of the pyrolytic reactor 100. A quench fluid (e.g., water) is sprayed into the pyrolytic reactor outlet stream 32, and the quench fluid prevents further reactions in the pyrolytic reactor outlet stream 32. The quench unit also removes particulates (e.g., soot) via line 42. Outlet stream 44 from the quench unit 40 may include acetylene, ethylene, hydrogen, methane, carbon monoxide, and carbon dioxide.

In the compression and acetylene recovery zone 50, the outlet stream 44 is compressed. The majority of the compressed gas is contacted with a solvent that absorbs acetylene, and the solvent and acetylene exit the acetylene recovery zone 50 via line 52. Suitable solvents include n-methyl-2-pyrrolidone, dimethylformamide, acetone, tetrahydrofuran, dimethylsulfoxide, monomethylamine, and combinations thereof. A minority of the compressed gas is conveyed via line 53. Gas that does not absorb in the solvent (e.g., hydrogen, methane, carbon monoxide, and carbon dioxide) exits the recovery zone 50 via line 58.

Streams 52 and 53 are combined in line 56 at the top of the hydrogenation reactor 60. In one non-limiting example configuration, stream 53 is the source of the hydrogen for the hydrogenation reaction. Alternatively hydrogen can be supplied or supplemented by other sources via line 53. In one non-limiting example configuration, the hydrogenation reactor 60 uses a liquid phase selective hydrogenation process (SHP) in which the solvent is n-methyl-2-pyrrolidone (NMP). The absorbed acetylene and solvent are contacted with a catalyst. In one embodiment, the catalyst contains at least one Group VIII metal on an inorganic support. In one embodiment, palladium is one of the Group VIII metals. In one embodiment, the catalyst also contains at least one metal from Group IB, IIB, IIIA. IVA, IA and VIIB. The acetylene is converted to ethylene in the hydrogenation reactor 60.

Stream 64 exits the hydrogenation reactor 60, and the stream 64 enters the product separator 70. The product separator 70 separates the desired product, ethylene, from any other components that may be present. The other components may include hydrogen, carbon dioxide, carbon monoxide, nitrogen, methane, or ethane as possible examples. The product separator 70 may comprise a conventional separation methods for recovery of ethylene such as cryogenic distillation, pressure-swing adsorption and membrane separation and may include additional selective hydrogenation reactors. In one example method, the product separator 70 provides an outlet stream 72, which may be a vapor, liquid, or combination, of ethylene, and an outlet stream 74 of ethane and byproducts, and an outlet stream 78 of hydrogen, carbon dioxide, carbon monoxide, nitrogen, and/or methane.

The outlet stream 78 of hydrogen, carbon dioxide, carbon monoxide, nitrogen, and/or methane is recycled to the pyrolytic reactor 100. In addition, line 58 which may include hydrogen, methane, carbon monoxide, and carbon dioxide can be fed to a carbon dioxide separator 80 to remove carbon dioxide. The carbon dioxide separator 80 can use an amine solvent, such as N-methyl diethanolamine, to absorb or otherwise separate CO₂ from the stream materials. A stripper can be subsequently used to strip the absorbed CO₂ from the amine solvent, permitting the reuse of the stripped amine solvent. One physical solvent process for capturing the CO₂ stream is UOP's Selexol process. Stream 82 from the carbon dioxide separator 80 may include hydrogen, methane, and carbon monoxide, and the stream 82 can be recycled to the pyrolytic reactor 100. Carbon dioxide exits the carbon dioxide separator 80 via line 84. Optionally, fuel gas can be removed from any of lines 58, 74, 78 or 82.

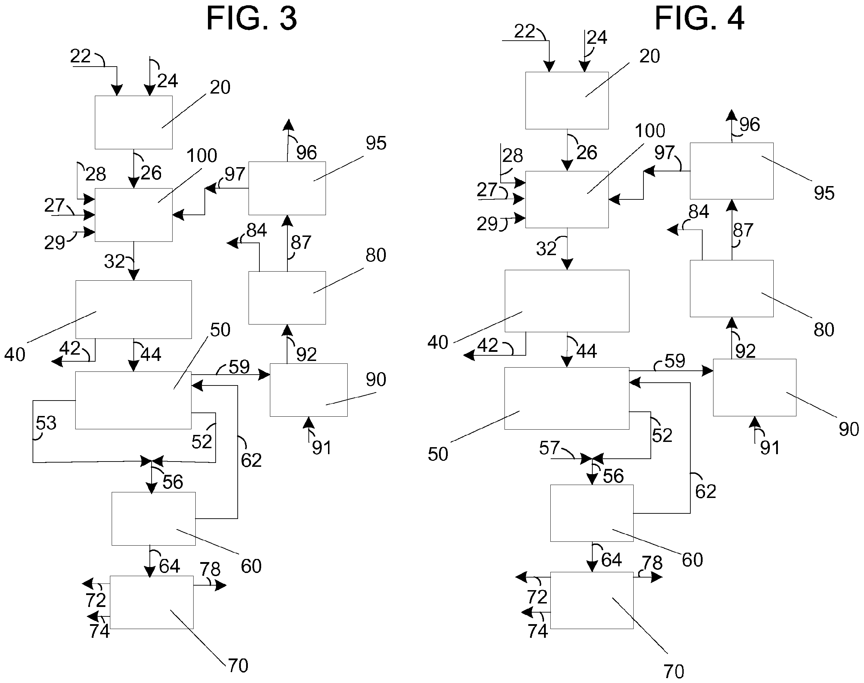

Turning now to Figure 3, there is shown another example process according to the invention for converting a light hydrocarbon (e.g., methane) to an alkyne (e.g., acetylene) and then converting the alkyne (e.g., acetylene) to an olefin (e.g., ethylene). First, the air separation unit 20 extracts oxygen from the air. The air separation unit 20 receives air via the air line 22, and generates the nitrogen rich stream 24 in which the oxygen content is less than that of air. The nitrogen rich stream 24 can be vented or used for other purposes. The air separation unit 0 also generates the oxygen rich stream 26 in which the oxygen content is greater than that of air. In one embodiment the oxygen content of stream 26 is greater than 80%. The air separation unit 20 can use a conventional pressure swing adsorption (PSA) process.

In the example process of Figure 3 methane is converted into acetylene in the pyrolytic reactor 100 (SWR) of Figure 1. The pyrolytic reactor 100 receives methane (CH₄) via feed lines 126 (see Fig. 1) that receive methane from methane line 28. The pyrolytic reactor 100 receives the oxidizer (oxygen) via oxygen rich stream 26. The pyrolytic reactor 100 receives the fuel (hydrogen) via hydrogen stream 27. A pyrolytic reactor outlet stream 32 produced by the pyrolytic reactor 100 may include acetylene, ethylene, hydrogen, methane, carbon monoxide, carbon dioxide, and carbon particulates.

The pyrolytic reactor outlet stream 32 is fed into the quench unit 40 to rapidly cool the reactive mixture in the pyrolytic reactor outlet stream 32. The quench unit 40 may be a separate unit, or it may be incorporated into the quenching zone 120 (see Fig. 1) of the pyrolytic reactor 100. A quench fluid (e.g., water) is sprayed into the pyrolytic reactor outlet stream 32, and the quench fluid prevents further reactions in the pyrolytic reactor outlet stream 32. The quench fluid also removes particulates (e.g., soot) via line 42. Outlet stream 44 from the quench unit 40 may include acetylene, ethylene, hydrogen, methane, carbon monoxide, and carbon dioxide.

In the compression and acetylene recovery zone 50, the outlet stream 44 is compressed. The majority of the compressed gas is combined with a solvent that absorbs acetylene, and the solvent and acetylene exit the acetylene recovery zone 50 via line 52. Suitable solvents include n-methyl-2-pyrrolidone, acetone, tetrahydrofuran, dimethylsulfoxide, monomethylamine, and combinations thereof. A minority of the compressed gas is conveyed via line 53. Gas that does not absorb in the solvent (e.g., hydrogen, methane, carbon monoxide, and carbon dioxide) exits the recovery zone 50 via line 59.

Streams 52 and 53 are combined in line 56 at the top of the hydrogenation reactor 60. Stream 53 is the source of the hydrogen for the hydrogenation reaction. In one non-limiting example configuration, the hydrogenation reactor 60 uses a liquid phase selective hydrogenation process (SHP) in which the solvent is n-methyl-2-pyrrolidone (NMP). The absorbed acetylene and solvent are contacted with a catalyst. In one embodiment the catalyst contains at least one Group VIII metal on an inorganic support. In one embodiment palladium is one of the Group VIII metals. In one embodiment palladium is one of the Group VIII metals. In one embodiment the catalyst also contains at least one metal from Group IB, IIB, IIIA. IVA, IA and VIIB. The acetylene is converted to ethylene in the hydrogenation reactor 60. The solvent can be recycled to the acetylene recovery zone 50 via line 62.

Stream 64 exits the hydrogenation reactor 60, and the stream 64 enters the product separator 70. The product separator 70 separates the desired product, ethylene, from any other components that may be present. The other components may include hydrogen, carbon dioxide, carbon monoxide, nitrogen, methane, or ethane as possible examples. The product separator 70 may comprise a conventional separation method such as cryogenic distillation, pressure-swing adsorption and membrane separation. In one example method, the product separator 70 provides an outlet stream 72, which may be a vapor, liquid, or combination, of ethylene, and an outlet stream 74 of ethane and byproducts, and an outlet stream 78 of hydrogen, carbon dioxide, carbon monoxide, nitrogen, and/or methane. The outlet stream 78 of hydrogen, carbon dioxide, carbon monoxide, nitrogen, and/or methane can be recycled to the pyrolytic reactor 100.

In addition, line 59, which may include hydrogen, methane, carbon monoxide, and carbon dioxide, is fed to a CO shift device 90. In the CO shift device 90, carbon monoxide is used for hydrogen generation according the chemical reaction CO + H₂O → H₂ + CO₂. In one embodiment, the water is supplied to the CO shift device 90 as steam via line 91. The CO shift conversion reaction may be a high temperature (HT) CO shift conversion (300°C to 450°C), or a low temperature (LT) CO shift conversion (180°C to 250°C), or a combination thereof. A catalyst in a fixed bed reactor can be used to get a suitable yield of hydrogen. In one example method in the CO shift device 90, low temperature CO shift conversion is used downstream of the high temperature CO shift conversion at an already reduced carbon monoxide content in the feed gas. CO shift catalysts are well known in the art and for example include iron-chromium oxide catalysts (Fe₃O₄/Cr₂O₃) catalysts for high temperature shift and copper oxide and zinc oxide supported on alumina for low temperature CO shift.

Stream 92, which may include hydrogen, methane, and carbon dioxide, exits the CO shift device 90 and is fed to a carbon dioxide separator 80 to remove carbon dioxide. Carbon dioxide exits the carbon dioxide separator 80 via line 84. Stream 87 from the carbon dioxide separator 80, which may include hydrogen and methane, is fed to a gas separator 95, which can use a conventional processes such as pressure swing adsorption (PSA) or membrane separation process. A hydrogen rich stream exits the gas separator 95 at line 96 which can be conveyed back to the pyrolytic reactor 100 via hydrogen stream 27. Methane exits the gas separator 95 at line 97 which can be conveyed back to the pyrolytic reactor 100 via feed lines 126 (see Fig. 1).

Referring now to Figure 4, there is shown yet another example process according to the invention for converting a light hydrocarbon (e.g., methane) to an alkyne (e.g., acetylene) and then converting the alkyne (e.g., acetylene) to an olefin (e.g., ethylene). The process of Figure 4 is similar to the process of Figure 3 (like reference numerals being used in Figure 4 to refer to like parts from Figure 3). However, in the process of Figure 4, line 53 of Figure 3 (which flows from acetylene recovery zone 50) is removed, and hydrogen is fed directly via a line 57 that is combined with stream 52 in line 56 at the top of the hydrogenation reactor 60. Line 57 is the source of the hydrogen for the hydrogenation reaction.

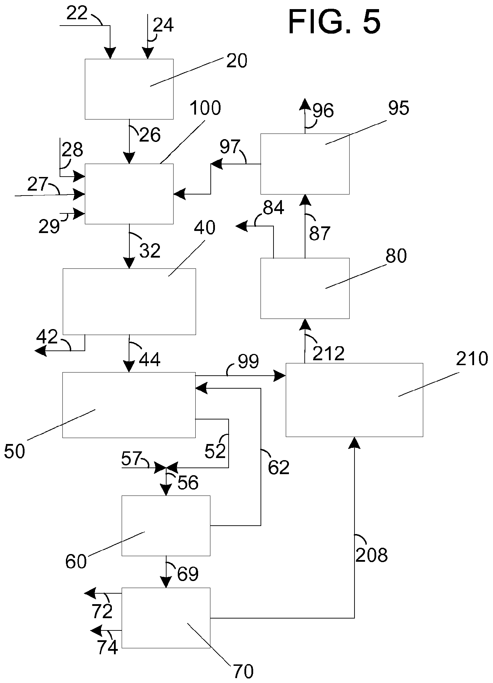

Turning now to Figure 5, there is shown still another example process according to the invention for converting a light hydrocarbon (e.g., methane) to an alkyne (e.g., acetylene) and then converting the alkyne (e.g., acetylene) to an olefin (e.g., ethylene). First, the air separation unit 20 extracts oxygen from the air. The air separation unit 20 receives air via the air line 22, and generates the nitrogen rich stream 24 in which the oxygen content is less than that of air. The air separation unit 20 also generates the oxygen rich stream 26 in which the oxygen content is greater than that of air.

In the example process of Figure 5, methane is converted into acetylene in the pyrolytic reactor 100 (SWR) of Figure 1. The pyrolytic reactor 100 receives methane (CH₄) via feed lines 126 (see Fig. 1) that receive methane from methane line 28. The pyrolytic reactor 100 receives the oxidizer (oxygen) via oxygen rich stream 26. The pyrolytic reactor 100 receives the fuel (hydrogen) via hydrogen stream 27. A pyrolytic reactor outlet stream 32 produced by the pyrolytic reactor 100 may include acetylene, ethylene, hydrogen, methane, carbon monoxide, carbon dioxide, and carbon particulates.

The pyrolytic reactor outlet stream 32 is fed into the quench unit 40 to rapidly cool the reactive mixture in the pyrolytic reactor outlet stream 32. The quench unit 40 may be a separate unit, or it may be incorporated into the quenching zone 120 (see Fig. 1) of the pyrolytic reactor 100. A quench fluid (e.g., water) is sprayed into the pyrolytic reactor outlet stream 32, and the quench fluid prevents further reactions in the pyrolytic reactor outlet stream 32. The quench fluid also removes particulates (e.g., soot) via line 42. Outlet stream 44 from the quench unit 40 may include acetylene, ethylene, hydrogen, methane, carbon monoxide, and carbon dioxide.

In the compression and acetylene recovery zone 50, the outlet stream 44 is compressed. The compressed gas is combined with a solvent that absorbs acetylene, and the solvent and acetylene exit the acetylene recovery zone 50 via line 52. Suitable solvents include n-methyl-2-pyrrolidone, acetone, tetrahydrofuran, dimethylsulfoxide, monomethylamine, and combinations thereof. Gas that does not absorb in the solvent (e.g., hydrogen, methane, carbon monoxide, and carbon dioxide) exits the recovery zone 50 via line 99. Hydrogen is fed directly via a line 57.

Streams 52 and 57 are combined in line 56 at the top of the hydrogenation reactor 60. Stream 57 is the source of the hydrogen for the hydrogenation reaction. In one non-limiting example configuration, the hydrogenation reactor 60 uses a liquid phase selective hydrogenation process (SHP) in which the solvent is n-methyl-2-pyrrolidone (NMP). The absorbed acetylene and solvent are contacted with a catalyst, such as a Group VIII metal. The acetylene is converted to ethylene in the hydrogenation reactor 60. The solvent can be recycled to the acetylene recovery zone 50 via line 62.

Stream 64 exits the hydrogenation reactor 60, and the stream 64 enters the product separator 70. The product separator 70 separates the desired product, ethylene, from any other components that may be present. The other components may include hydrogen, carbon dioxide, carbon monoxide, nitrogen, methane, or ethane as possible examples. The product separator 70 may comprise a conventional separation method such as cryogenic distillation, pressure-swing adsorption and membrane separation. In one example method, the product separator 70 provides an outlet stream 72, which may be a vapor, liquid, or combination, of ethylene, and an outlet stream 74 of ethane and byproducts, and an outlet stream 208 of hydrogen, carbon dioxide, carbon monoxide, nitrogen, and/or methane.

The outlet stream 208 of hydrogen, carbon dioxide, carbon monoxide, nitrogen, and methane, and line 99, which can include hydrogen, methane, carbon monoxide, and carbon dioxide are fed to a carbon dioxide conversion and methanation zone 210.

In the carbon dioxide conversion and methanation zone 210, a reverse water gas shift catalyst facilitates the reduction of carbon dioxide to carbon monoxide in accordance with a reverse water gas shift reaction as follows: CO₂+ H₂ → CO + H₂O. A temperature of 200°C to 500°C, and a pressure of 100 to 5,000 kPa are suitable for the reverse water gas shift reaction. Water produced in the reverse water gas shift reaction can be removed from the carbon dioxide conversion and methanation zone 210.

Non-limiting examples of reverse water gas shift catalysts are solid acid catalysts including FAU, BEA, MWW, UZM-4, UZM-5, UZM-8, MOR, MEI, MTW, SPA and cesium (Cs) salts of heteropoly acid. FAU, BEA, MWW, BPH, UFI, MOR, MEI, MTW are 3-letter codes representing the framework types and are assigned by Structural Commission of International Zeolite Association. BEA or zeolite beta is a microporous alumino-silicate that has three intersecting 12-ring channels. UZM-4 is a crystalline alumino-silicate as described in U.S. Pat. No. 6,419,895. UZM-4M is a modified form of UZM-4 using a process described in U.S. Pat. No. 6,776,975. UZM-5 is a crystalline alumino-silicate as set forth in U.S. Pat. No. 6,613,302. UZM-8 is a crystalline zeolite containing a layered framework of aluminum oxide and silicon dioxide tetrahedral units as disclosed in U.S. Pat. No. 6,756,030. Mordenite is a crystalline zeolite having one 12-ring channel with two intersecting 8-R channels. MTW is a microporous alumino-silicate that has one 12-ring channel as disclosed in U.S. Pat. No. 6,872,866. Solid phosphoric acid (SPA) is phosphoric acid supported on silica phosphate.

After the reverse water gas shift reaction in the carbon dioxide conversion and methanation zone 210, a methanation catalyst is used to remove carbon monoxide by reaction with hydrogen to form methane and water under methanation conditions. The methanation reaction is CO + 3H₂ → CH₄ + H₂U. The methanation catalyst can also convert remaining carbon dioxide via the reaction CO₂ + 4H₂ → CH₄ + 2H₂O. Generally, the methanation catalyst includes nickel, cobalt, or ruthenium, preferably nickel, and can be provided in any suitable manner, such as a packed bed, a fluidized bed, a coated heat exchanger tube, or a slurry catalyst mixture. Methanation conditions can include a temperature of 200°C to 400°C, and a pressure of 600 to 4,500 kPa. Water produced in the methanation reaction can be removed from the carbon dioxide conversion and methanation zone 210.

Stream 212, which may include hydrogen, methane, and small amounts of unreacted carbon dioxide, exits the carbon dioxide conversion and methanation zone 210 and is fed to a carbon dioxide separator 80 to remove carbon dioxide. Carbon dioxide exits the carbon dioxide separator 80 via line 84. Stream 87 from the carbon dioxide separator 80, which may include hydrogen and methane, is fed to a gas separator 95, which can use a conventional pressure swing adsorption (PSA) process. Hydrogen exits the gas separator 95 at line 96 which can be conveyed back to the pyrolytic reactor 100 via hydrogen stream 27. Methane exits the gas separator 95 at line 97 which can be conveyed back to the pyrolytic reactor 100 via feed lines 126 (see Fig. 1).

Table 1 below provides the methane to ethylene overall process material flow estimates for the processes depicted in Figures 2-5. The amount of estimated required methane is based on the chemical requirements that sustain the reactor zone combustion and the chemical conversion.

**Table 1**

| Case ID -> | Case-0 | Case-1 | Case-2 | Case- 3 | Case-4 | Case-5 | Case-6 |
|---|---|---|---|---|---|---|---|
| Process -> | Prior Art | Fig. 2 | Fig. 3 | Fig. 3 | Fig. 4 | Fig. 4 | Fig. 5 |
| | | | | | | | |

| INPUT | | | | | | | |
|---|---|---|---|---|---|---|---|
| CH₄ - Burn | 0.0 | 0.0 | 377.5 | 379.1 | 370.2 | 0.0 | 376.5 |
| CH₄ | 1562.0 | 1030.1 | 815.6 | 693.2 | 676.8 | 787.8 | 352.0 |
| O₂ | 1895.2 | 1112.3 | 1109.4 | 1114.2 | 1087.9 | 1092.5 | 1106.4 |
| H₂O | 45.0 | 0.0 | 0.0 | 0.0 | 0.0 | 0.0 | 0.0 |
| Total | 3502.1 | 2142.4 | 2302.5 | 2186.4 | 2134.8 | 1880.3 | 1834.8 |
| | | | | | | | |

| OUTPUT | | | | | | | |
|---|---|---|---|---|---|---|---|
| Fuel Gas | 1707.9 | 463.0 | 283.5 | 0.0 | 0.0 | 0.0 | 0.0 |
| C₂H₄ Finished | 500.0 | 500.0 | 500.0 | 500.0 | 500.0 | 500.0 | 500.0 |
| H₂ | 0.0 | 0.0 | 111.6 | 176.7 | 170.6 | 35.5 | 1.8 |
| H₂O | 1006.0 | 763.6 | 255.5 | 204.8 | 185.5 | 804.7 | 969.8 |
| CO₂ | 288.2 | 415.8 | 1151.9 | 1305.0 | 1278.8 | 540.1 | 363.2 |
| | | | | | | | |
| Total | 3502.1 | 2142.4 | 2302.5 | 2186.4 | 2134.8 | 1880.3 | 1834.8 |
| | | | | | | | |
| % C Efficiency | 36.6 | 55.5 | 47.9 | 53.3 | 54.6 | 72.5 | 78.4 |

Case 0 is prior art where methane is converted to acetylene in a partial oxidation reactor. The product gas containing acetylene and hydrogen is separated to produce a feed to a selective hydrogenation reactor that selectively hydrogenates acetylene with hydrogen to produce ethylene. The resulting ethylene can be separated from other products and the result is the yield given in Table 1. If part of the product CO in the fuel gas is burned in a later use (for example used as a fuel) that will result in increased carbon dioxide emissions.

Case 1 needs much more methane to produce the same amount of ethylene compared to Cases 2-6. Case-6 has the lowest CO₂ emission. The CO₂ conversion zone in this configuration contains a solid acid catalyst that is capable of converting CO₂ to CO in the presence of hydrogen and other hydrocarbons. The methanation section converts the CO into methane by utilizing the hydrogen in the gas. The gas product from this combined zone contains reduced CO₂ and CO plus all hydrocarbons, including the methane that was produced from CO / CO₂. The CO₂ is removed first. The PSA separates the hydrogen and the hydrocarbon stream that also includes the remainder small amount of CO. The hydrocarbon stream is fed to the reactor's hydrocarbon feed section. For the combustor section, methane is used. In this configuration, Case-6 increases the carbon efficiency by 150% compared to its counterpart Case-3. Case-6 also has much less CO₂ emissions compared to Case-3. Cases 2-6 produce high purity hydrogen as a byproduct. Thus, the processes of Figures 3-6 have carbon efficiencies of greater than 40%, or greater than 50%, or greater than 60%, or greater than 70%, wherein carbon efficiency (%) = amount of carbon in product / total carbon present in reactants x 100.

Thus, the invention provides high efficiency processes for producing olefins, alkynes, and hydrogen co-production from light hydrocarbons. In one embodiment, the high efficiency processes can produce C₁ to C₄ olefins and C₁ to C₄ alkynes from light hydrocarbons (C₁ to C₄ alkanes, i.e., methane, ethane, propane and butane).

Although the invention has been described in considerable detail with reference to certain embodiments, one skilled in the art will appreciate that the present invention can be practiced by other than the described embodiments, which have been presented for purposes of illustration and not of limitation. Therefore, the scope of the appended claims should not be limited to the description of the embodiments contained herein.

In one embodiment, the invention relates to A method of making alkenes and alkynes, the method comprising: combusting a fuel and an oxidizer in a combustion zone of a pyrolytic reactor to create a combustion gas stream; transitioning a velocity of the combustion gas stream from subsonic to supersonic in an expansion zone of the pyrolytic reactor; injecting a light hydrocarbon into the supersonic combustion gas stream to create a mixed stream including the light hydrocarbon; transitioning the velocity of the mixed stream from supersonic to subsonic in a reaction zone of the pyrolytic reactor to produce an alkyne; and catalytically hydrogenating the alkyne in a hydrogenation zone to produce an alkene. In one embodiment the fuel is hydrogen, the oxidizer is oxygen, the light hydrocarbon is methane, the alkyne is acetylene, and the alkene is ethylene. The transitioning the velocity of the mixed stream from supersonic to subsonic forms a shockwave resulting in an increase in pressure and temperature of the mixed stream. In one embodiment a first temperature of the mixed stream immediately upstream of the shock wave is 1500 K to 2300 K, and a second temperature of the mixed stream is 1600 K to 2800 K immediately downstream of the shockwave. The invention may further involve introducing a product stream including the alkyne from the pyrolytic reactor into a hydrogenation reactor for catalytically hydrogenating the alkyne to produce the alkene; introducing a treated product stream including the alkene into a product separator; separating the alkene from the treated product stream in the product separator to create a recyclable stream including at least one of hydrogen and methane; and directing the recyclable stream into the pyrolytic reactor. The method may further comprise separating a recyclable stream from the hydrogenation zone, the recyclable stream including carbon dioxide; treating the recyclable stream to remove carbon dioxide; and directing the treated recyclable stream into the pyrolytic reactor. The method may further comprise separating a recyclable stream from the hydrogenation zone, the recyclable stream including carbon monoxide; treating the recyclable stream to convert at least a portion of the carbon monoxide to hydrogen; and directing the treated recyclable stream into the pyrolytic reactor. The method may further comprise separating a recyclable stream from the hydrogenation zone, the recyclable stream including carbon monoxide and carbon dioxide; treating the recyclable stream to convert at least a portion of the carbon monoxide to hydrogen; treating the recyclable stream to remove carbon dioxide; and directing the treated recyclable stream into the pyrolytic reactor. The method may further comprise separating a recyclable stream from the hydrogenation zone, the recyclable stream including hydrogen and methane; treating the recyclable stream to separate the hydrogen and the methane and to create a hydrogen stream and a methane stream; directing the hydrogen stream as the fuel into the combustion zone of the pyrolytic reactor; and directing the methane stream as the light hydrocarbon into the combustion gas stream in the pyrolytic reactor. The method may further comprise separating a recyclable stream from the hydrogenation zone, the recyclable stream including carbon monoxide; treating the recyclable stream to convert at least a portion of the carbon monoxide to hydrogen; treating the recyclable stream to separate the hydrogen and create a hydrogen stream; and directing the hydrogen stream as the fuel into the combustion zone of the pyrolytic reactor. The method may further comprise separating a recyclable stream from the hydrogenation zone, the recyclable stream including carbon dioxide; converting at least a portion of the carbon dioxide in the recyclable stream to methane in a carbon dioxide conversion and methanation zone; and directing the methane as the light hydrocarbon into the combustion gas stream in the pyrolytic reactor. The method may involve wherein the converting at least a portion of the carbon dioxide in the recyclable stream to methane in a carbon dioxide conversion and methanation zone may comprise reducing the carbon dioxide in the recyclable stream to carbon monoxide, and reacting the carbon monoxide with hydrogen to form the methane. The method may further comprise separating a recyclable stream from the hydrogenation zone, the recyclable stream including carbon dioxide; converting at least a portion of the carbon dioxide in the recyclable stream to methane in a carbon dioxide conversion and methanation zone; treating the recyclable stream to remove carbon dioxide; treating the recyclable stream to separate the hydrogen and the methane and to create a hydrogen stream and a methane stream; directing the hydrogen stream as the fuel into the combustion zone of the pyrolytic reactor; and directing the methane stream as the light hydrocarbon into the combustion gas stream in the pyrolytic reactor. The method wherein the catalytically hydrogenating comprises introducing a product stream including the alkyne from the pyrolytic reactor into a hydrogenation reactor for catalytically hydrogenating the alkyne to produce the alkene; introducing a treated product stream including the alkene into a product separator; separating the alkene from the treated product stream in the product separator to create a recyclable stream including carbon dioxide; and the method further comprises: converting at least a portion of the carbon dioxide in the recyclable stream to methane in a carbon dioxide conversion and methanation zone; and directing the methane as the light hydrocarbon into the combustion gas stream in the pyrolytic reactor.

In another embodiment the invention involves a method of making alkenes and alkynes, the method comprising: performing pyrolysis of a light hydrocarbon in the presence of oxygen in a reaction zone at a temperature and pressure suitable to produce an alkyne and carbon monoxide; catalytically hydrogenating the alkyne in a hydrogenation zone to produce an alkene; directing the carbon monoxide to a CO shift device; converting at least a portion of the carbon monoxide to hydrogen in the CO shift device to produce a stream including the hydrogen; and directing the stream including the hydrogen into the reaction zone. The stream may include carbon dioxide, and the method may further comprise removing carbon dioxide from the stream. The method may further comprise: treating the stream to separate out other gases before directing the stream including the hydrogen into the reaction zone.

In another embodiment the invention relates to a method of making alkenes and alkynes, the method comprising: performing pyrolysis of a light hydrocarbon in the presence of oxygen in a reaction zone at a temperature and pressure suitable to produce an alkyne and carbon dioxide; catalytically hydrogenating the alkyne in a hydrogenation zone to produce an alkene; converting at least a portion of the carbon dioxide to methane in a carbon dioxide conversion and methanation zone; and directing a stream including the methane from the carbon dioxide conversion and methanation zone into the reaction zone. The stream may include carbon dioxide, and the method further comprises removing carbon dioxide from the stream. The method may include treating the stream to separate out other gases before directing the stream including the methane into the reaction zone.

## Claims

1. A method of making alkenes and alkynes, the method comprising:
(a) combusting a fuel and an oxidizer in a combustion zone of a pyrolytic reactor to create a combustion gas stream;
(b) transitioning a velocity of the combustion gas stream from subsonic to supersonic in an expansion zone of the pyrolytic reactor;
(c) injecting a light hydrocarbon into the supersonic combustion gas stream to create a mixed stream including the light hydrocarbon;
(d) transitioning the velocity of the mixed stream from supersonic to subsonic in a reaction zone of the pyrolytic reactor to produce an alkyne;
(e) catalytically hydrogenating the alkyne in a hydrogenation zone to produce an alkene; and
(f) recycling a stream from the hydrogenation zone, the recyclable stream including carbon monoxide and/or carbon dioxide.

2. The method of claim 1 wherein:
the fuel is hydrogen,
the oxidizer is oxygen,
the light hydrocarbon is methane,
the alkyne is acetylene,
the alkene is ethylene, and
wherein transitioning the velocity of the mixed stream from supersonic to subsonic in step (d) forms a shockwave resulting in an increase in pressure and temperature of the mixed stream.

3. The method of claim 2 wherein:
a first temperature of the mixed stream immediately upstream of the shock wave is 1500 K to 2300 K, and
a second temperature of the mixed stream is 1600 K to 2800 K immediately downstream of the shockwave.

4. The method of claim 1 further comprising:
(f) separating a recyclable stream from the hydrogenation zone, the recyclable stream including carbon monoxide;
(g) treating the recyclable stream to: (i) convert at least a portion of the carbon monoxide to hydrogen, (ii) remove carbon dioxide, or (iii) both convert at least a portion of the carbon monoxide to hydrogen and remove carbon dioxide; and
(h) directing the treated recyclable stream into the pyrolytic reactor.

5. The method of claim 1 further comprising:
(f) separating a recyclable stream from the hydrogenation zone, the recyclable stream including carbon monoxide;
(g) treating the recyclable stream to convert at least a portion of the carbon monoxide to hydrogen;
(h) treating the recyclable stream to separate the hydrogen and create a hydrogen stream; and
(i) directing the hydrogen stream as the fuel into the combustion zone of the pyrolytic reactor.

6. The method of claim 1 further comprising:
(f) separating a recyclable stream from the hydrogenation zone, the recyclable stream including carbon dioxide;
(g) converting at least a portion of the carbon dioxide in the recyclable stream to methane in a carbon dioxide conversion and methanation zone; and
(h) directing the methane as the light hydrocarbon into the combustion gas stream in the pyrolytic reactor.

7. The method of claim 1 further comprising:
(f) separating a recyclable stream from the hydrogenation zone, the recyclable stream including carbon dioxide;
(g) converting at least a portion of the carbon dioxide in the recyclable stream to methane in a carbon dioxide conversion and methanation zone;
(h) treating the recyclable stream to remove carbon dioxide;
(i) treating the recyclable stream to separate the hydrogen and the methane and to create a hydrogen stream and a methane stream;
(j) directing the hydrogen stream as the fuel into the combustion zone of the pyrolytic reactor; and
(k) directing the methane stream as the light hydrocarbon into the combustion gas stream in the pyrolytic reactor.

8. The method of claim 1 wherein:
step (e) comprises
(i) introducing a product stream including the alkyne from the pyrolytic reactor into a hydrogenation reactor for catalytically hydrogenating the alkyne to produce the alkene;
(ii) introducing a treated product stream including the alkene into a product separator;
(iii) separating the alkene from the treated product stream in the product separator to create a recyclable stream including carbon dioxide; and
the method further comprises:
(g) converting at least a portion of the carbon dioxide in the recyclable stream to methane in a carbon dioxide conversion and methanation zone; and
(h) directing the methane as the light hydrocarbon into the combustion gas stream in the pyrolytic reactor.

## Patentansprüche

1. Verfahren zum Herstellen von Alkenen und Alkinen, das Verfahren umfassend:
(a) Verbrennen eines Brennstoffs und eines Oxidationsmittels in einer Verbrennungszone eines pyrolytischen Reaktors, um einen Verbrennungsgasstrom zu erzeugen;
(b) Überführen einer Geschwindigkeit des Verbrennungsgasstroms von Unterschall zu Überschall in einer Expansionszone des pyrolytischen Reaktors;
(c) Injizieren eines leichten Kohlenwasserstoffes in den Überschallverbrennungsgasstrom, um einen gemischten Strom zu erzeugen, der den leichten Kohlenwasserstoff enthält;
(d) Überführen der Geschwindigkeit des gemischten Stroms von Überschall zu Unterschall in einer Reaktionszone des pyrolytischen Reaktors, um ein Alkin herzustellen;
(e) katalytisches Hydrieren des Alkins in einer Hydrierungszone, um ein Alken herzustellen; und
(f) Rückführen eines Stroms von der Hydrierungszone, wobei der rückführbare Strom Kohlenmonoxid und/oder Kohlendioxid enthält.

2. Verfahren nach Anspruch 1, wobei:
der Brennstoff Wasserstoff ist,
das Oxidationsmittel Sauerstoff ist,
der leichte Kohlenwasserstoff Methan ist,
das Alkin Acetylen ist,
das Alken Ethylen ist und
wobei das Überführen der Geschwindigkeit des gemischten Stroms von Überschall zu Unterschall in Schritt (d) eine Stoßwelle bildet, die zu einem Anstieg des Drucks und der Temperatur des gemischten Stroms führt.

3. Verfahren nach Anspruch 2, wobei:
eine erste Temperatur des gemischten Stroms unmittelbar stromaufwärts der Stoßwelle 1500 K bis 2300 K beträgt und
wobei eine zweite Temperatur des gemischten Stroms 1600 K bis 2800 K unmittelbar stromabwärts der Stoßwelle beträgt.

4. Verfahren nach Anspruch 1, ferner umfassend:
(f) Trennen eines rückführbaren Stroms von der Hydrierungszone, wobei der rückführbare Strom Kohlenmonoxid enthält;
(g) Behandeln des rückführbaren Stroms zum: (i) Umwandeln von mindestens einem Teil des Kohlenmonoxids in Wasserstoff, (ii) Entfernen des Kohlendioxids oder (iii) sowohl Umwandeln von mindestens einem Teil des Kohlenmonoxids in Wasserstoff als auch Entfernen des Kohlendioxids; und
(h) Leiten des behandelten rückführbaren Stroms in den pyrolytischen Reaktor.

5. Verfahren nach Anspruch 1, ferner umfassend:
(f) Trennen eines rückführbaren Stroms von der Hydrierungszone, wobei der rückführbare Strom Kohlenmonoxid enthält;
(g) Behandeln des rückführbaren Stroms, um mindestens einen Teil des Kohlenmonoxids in Wasserstoff umzuwandeln;
(h) Behandeln des rückführbaren Stroms, um den Wasserstoff abzuscheiden und um einen Wasserstoffstrom zu erzeugen; und
(i) Leiten des Wasserstoffstroms als Brennstoff in die Verbrennungszone des pyrolytischen Reaktors.

6. Verfahren nach Anspruch 1, ferner umfassend:
(f) Trennen eines rückführbaren Stroms von der Hydrierungszone, wobei der rückführbare Strom Kohlendioxid enthält;
(g) Umwandeln von mindestens einem Teil des Kohlendioxids im rückführbaren Strom in Methan in einer Kohlendioxidumwandlungs- und Methanisierungszone; und
(h) Leiten des Methans als den leichten Kohlenwasserstoff in den Verbrennungsgasstrom im pyrolytischen Reaktor.

7. Verfahren nach Anspruch 1, ferner umfassend:
(f) Trennen eines rückführbaren Stroms von der Hydrierungszone, wobei der rückführbare Strom Kohlendioxid enthält;
(g) Umwandeln von mindestens einem Teil des Kohlendioxids im rückführbaren Strom in Methan in einer Kohlendioxidumwandlungs- und Methanisierungszone;
(h) Behandeln des rückführbaren Stroms, um Kohlendioxid zu entfernen;
(i) Behandeln des rückführbaren Stroms, um den Wasserstoff und das Methan abzuscheiden und um einen Wasserstoffstrom und einen Methanstrom zu erzeugen;
(j) Leiten des Wasserstoffstroms als Brennstoff in die Verbrennungszone des pyrolytischen Reaktors; und
(k) Leiten des Methanstroms als den leichten Kohlenwasserstoff in den Verbrennungsgasstrom im pyrolytischen Reaktor.

8. Verfahren nach Anspruch 1, wobei:
der Schritt (e) Folgendes umfasst
(i) Einführen eines Produktstroms, der das Alkin enthält, vom pyrolytischen Reaktor in einen Hydrierungsreaktor zum katalytischen Hydrieren des Alkins, um das Alken herzustellen;
(ii) Einführen eines behandelten Produktstroms, der das Alken enthält, in einen Produktabscheider;
(iii) Abscheiden des Alkens vom behandelten Produktstrom im Produktabscheider, um einen rückführbaren Strom zu erzeugen, der Kohlendioxid enthält; und
wobei das Verfahren ferner umfasst:
(g) Umwandeln von mindestens einem Teil des Kohlendioxids im rückführbaren Strom in Methan in einer Kohlendioxidumwandlungs- und Methanisierungszone; und
(h) Leiten des Methans als den leichten Kohlenwasserstoff in den Verbrennungsgasstrom im pyrolytischen Reaktor.

## Revendications

1. Procédé de fabrication d'alcènes et d'alcynes, le procédé comprenant :
(a) la combustion d'un combustible avec un oxydant dans une zone de combustion d'un réacteur pyrolytique pour créer un flux de gaz de combustion ;
(b) le passage d'une vitesse du flux de gaz de combustion de subsonique à supersonique dans une zone d'expansion du réacteur pyrolytique ;
(c) l'injection d'un hydrocarbure léger dans le flux de gaz de combustion supersonique pour créer un flux mixte contenant l'hydrocarbure léger ;
(d) le passage de la vitesse du flux mixte de supersonique à subsonique dans une zone de réaction du réacteur pyrolytique pour produire un alcyne ;
(e) l'hydrogénation catalytique de l'alcyne dans une zone d'hydrogénation pour produire un alcène ; et
(f) le recyclage d'un flux à partir de la zone d'hydrogénation, le flux recyclable contenant du monoxyde de carbone et/ou du dioxyde de carbone.

2. Procédé selon la revendication 1, dans lequel :
le combustible est l'hydrogène,
l'oxydant est l'oxygène,
l'hydrocarbure léger est le méthane,
l'alcyne est l'acétylène,
l'alcène est l'éthylène et
la transition de la vitesse du flux mixte de supersonique à subsonique à l'étape (d) formant une onde de choc entraînant une augmentation de la pression et de la température du flux mixte.

3. Procédé selon la revendication 2, dans lequel :
une première température du flux mixte immédiatement en amont de l'onde de choc est de 1 500 K à 2 300 K et
une deuxième température du flux mixte est de 1 600 K à 2 800 K immédiatement en aval de l'onde de choc.

4. Procédé selon la revendication 1, comprenant en outre :
(f) la séparation d'un flux recyclable à partir de la zone d'hydrogénation, le flux recyclable contenant du monoxyde de carbone ;
(g) le traitement du flux recyclable pour : (i) convertir au moins une partie du monoxyde de carbone en hydrogène, (ii) éliminer le dioxyde de carbone ou (iii) convertir au moins une partie du monoxyde de carbone en hydrogène et éliminer à la fois le dioxyde de carbone ; et
(h) diriger le flux recyclable traité dans le réacteur pyrolytique.

5. Procédé selon la revendication 1, comprenant en outre :
(f) la séparation d'un flux recyclable à partir de la zone d'hydrogénation, le flux recyclable contenant du monoxyde de carbone ;
(g) le traitement du flux recyclable pour convertir au moins une partie du monoxyde de carbone en hydrogène ;
(h) le traitement du flux recyclable pour séparer l'hydrogène et créer un flux d'hydrogène ; et
(i) la direction du flux d'hydrogène comme combustible dans la zone de combustion du réacteur pyrolytique.

6. Procédé selon la revendication 1, comprenant en outre :
(f) la séparation d'un flux recyclable à partir de la zone d'hydrogénation, le flux recyclable contenant du dioxyde de carbone ;
(g) la conversion d'au moins une partie du dioxyde de carbone dans le flux recyclable en méthane dans une zone de conversion et de méthanation du dioxyde de carbone ; et
(h) la direction du méthane sous forme d'hydrocarbure léger dans le flux de gaz de combustion dans le réacteur pyrolytique.

7. Procédé selon la revendication 1, comprenant en outre :
(f) la séparation d'un flux recyclable à partir de la zone d'hydrogénation, le flux recyclable contenant du dioxyde de carbone ;
(g) la conversion d'au moins une partie du dioxyde de carbone dans le flux recyclable en méthane dans une zone de conversion et de méthanation du dioxyde de carbone ;
(h) le traitement du flux recyclable pour éliminer le dioxyde de carbone ;
(i) le traitement du flux recyclable pour séparer l'hydrogène et le méthane et pour créer un flux d'hydrogène et un flux de méthane ;
(j) la direction du flux d'hydrogène sous forme de combustible dans la zone de combustion du réacteur pyrolytique ; et
(k) la direction du flux de méthane sous forme d'hydrocarbure léger dans le flux de gaz de combustion dans le réacteur pyrolytique.

8. Procédé selon la revendication 1, dans lequel :
l'étape (e) comprend
(i) l'introduction d'un flux de produits contenant l'alcyne à partir du réacteur pyrolytique dans un réacteur d'hydrogénation pour l'hydrogénation catalytique de l'alcyne pour produire l'alcène ;
(ii) l'introduction d'un flux de produits traité contenant l'alcène vers un séparateur de produits ;
(iii) la séparation de l'alcène à partir du flux de produits traité dans le séparateur de produits pour créer un flux recyclable contenant du dioxyde de carbone ; et
le procédé comprend en outre :
(g) la conversion d'au moins une partie du dioxyde de carbone dans le flux recyclable en méthane dans une zone de conversion et de méthanation du dioxyde de carbone ; et
(h) la direction du méthane sous forme d'hydrocarbure léger dans le flux de gaz de combustion dans le réacteur pyrolytique.
